# EUROPÄISCHE PATENTSCHRIFT

(11) **EP 1 866 013 B1**
(45) Veröffentlichungstag und Bekanntmachung des Hinweises auf die Patenterteilung: **03.10.2018**
(21) Anmeldenummer: 05745480.3
(22) Anmeldetag: 02.05.2005
(51) Int. Cl.: A61M 5/32

(54) **FOLIENBASIERTER SCHUTZMECHANISMUS**
FILM-BASED PROTECTIVE MECHANISM
MECANISME DE PROTECTION COMPRENANT UNE FEUILLE

(30) Priorität: 06.04.2005 DE 102005015801
(43) Veröffentlichungstag der Anmeldung: 19.12.2007
(73) Patentinhaber: Schreiner Group GmbH & Co. KG, 85764 Oberschleissheim (DE)
(72) Erfinder: BAUSS, Markus, 84435 Lengdorf (DE); LICHA, Robert, 82024 Taufkirchen (DE); MOOSHEIMER, Ulrich, 85411 Hohenkammer (DE); UNGLERT, Robert, 80639 München (DE)
(74) Vertreter: Epping - Hermann - Fischer
(86) Internationale Anmeldenummer: PCT/EP2005/004735
(87) Internationale Veröffentlichungsnummer: WO 2006/105807

(56) Entgegenhaltungen:
- WO-A-99/36114
- US-A- 4 758 229
- US-A- 5 383 862
- US-A- 5 462 531
- US-A1- 2002 065 488

## Beschreibung

Die vorliegende Erfindung betrifft einen Schutzmechanismus für Spritzen, Nadeln oder andere spitze oder scharfe Gegenstände aufweisende Vorrichtungen. Speziell betrifft sie einen Schutzmechanismus für derartige Vorrichtungen im medizinisch-pharmazeutischen Anwendungsbereich.

Zahlreiche Verletzungen im klinischen oder anderweitig medizinischen Bereich entstehen durch versehentlichen Kontakt mit scharfen und/oder spitzen Behandlungsvorrichtungen, in erster Linie Spritzennadeln oder Skalpellen. Derartige Verletzungen sind für die Betroffenen vor allem deshalb so gefährlich, weil hierdurch oftmals Infektionsrisiken entstehen, beispielsweise bei direktem Eindringen von Körperflüssigkeiten beispielsweise mit Hepatitis oder dem HI-Virus infizierter Patienten in die Blutbahn des Verletzten.

Daher ist es wünschenswert, derartige medizinische Geräte mit Schutzmechanismen auszustatten, die einen Schutz vor Verletzungen bieten.

An solche Schutzmechanismen bestehen folgende Anforderungen:
- Eine zufällige Kollision zwischen Spitze, Nadel und/oder Klinge und Anwender (Verletzung durch Stich oder Schnitt) muss ausgeschlossen sein.
- Die Hände sollten stets hinter der Spitze, Nadel und/oder Klinge bleiben.
- Der Schutzmechanismus sollte ein integraler Bestandteil der Vorrichtung sein, die die Spitze, Nadel und/oder Klinge aufweist.
- Er sollte sowohl vor als auch kurz nach der Anwendung der Vorrichtung (beispielsweise einer Injektion) als auch im Entsorgungsprozess (Abfall) wirksam sein.
- Schutzmechanismen sollten ohne Unterweisung einfach benutzbar sein und im Idealfall automatisch und einhändig bedienbar funktionieren.
- Während der Anwendung der Vorrichtung (beispielsweise einer Injektion), also während der Schutz zwangsläufig deaktiviert ist, darf der Schutzmechanismus dem Anwender nicht im Weg sein.
- Er sollte einen klaren, visuellen Hinweis auf seinen Zustand (Schutz unwirksam/wirksam) geben.
- Seine Aktivierung sollte idealerweise irreversibel ausgeführt sein, damit der Schutz nicht aufgehoben werden kann.
- Der Schutzmechanismus sollte zuverlässig und umweltverträglich sowie preiswert sein.

Zum Schutz von scharfen Nadeln ist eine Vielzahl von Mechanismen bekannt, die eine mehr oder weniger ideale Umsetzung der obigen Anforderungen sind. Beispielsweise offenbaren US 4 735 618 und US 6 796 968 Nadelhüllen, die mit Hilfe von Scharniergelenkmechanismen nach Verabreichen der Injektion über die Nadel geschoben werden. In US 5 879 337 wird eine Nadelkappe beschrieben, die entlang der Nadel gleitend zur Spitze geschoben und mit Hilfe eines Haltefadens so gehalten wird, dass sie nicht von der Nadelspitze herunter gleitet. Auch Federelemente sind hier beschrieben.

Die Schutzmechanismen sind üblicherweise aus Plastikspritzgussteilen und ggf. Federelementen aufgebaute einfache Mechaniken, die während der Injektion nicht behindern, jedoch nach der Injektion mit einfachen Auslöseprinzipien aktiviert werden können und scharfe Nadeln fortan schützend umschließen.

Die Schutzvorrichtungen können bereits in Spritzen oder Kanülen integriert sein oder Zusatzteile sein, die nachträglich auf Kanülen oder Spritzen befestigt werden können.

Neben einfachen per Hand verschieb-, klapp- oder klemmbaren Vorrichtungen sind auch komfortablere Spritzenprotektoren bekannt, die beispielsweise am Ende einer Injektion (Spritzenkolben in Endposition) automatisch auslösen und die Kanülen in eine gesicherte Position oder eine Schutzröhre in eine Barriereposition bringen.

Der größte Nachteil der bekannten Spritzenprotektoren sind deren mechanische Komplexität und Materialeigenschaften, die signifikante Zusatzkosten zum Spritzenkörper ergeben.

Ein weiterer Nachteil der bekannten Spritzenprotektoren ist es, dass diese üblicherweise nachträglich mit der Spritze oder Kanüle verbunden werden müssen, also einen zusätzlichen Fertigungsschritt erfordern.

US 5 462 531 zeigt einen Schutzmechanismus für einen Spritzenkörper mit einer verletzungsgefährlichen Nadel, wobei ein Verbindungsbereich der Schutzvorrichtung als Folie ausgebildet und an dem Spritzenkörper befestigt ist. Aufgabe der Erfindung ist es, einfach und zuverlässig arbeitende Schutzmechanismen aus preiswerten Materialien bereit zu stellen. Eine zweite Aufgabe besteht darin, Schutzmechanismen bereit zu stellen, die nicht in einem separaten Verfahrensschritt mit der Vorrichtung, die eine Spritze, Nadel oder andere spitze oder scharfe Gegenstände aufweist, verbunden werden müssen.

Die Aufgabe der Erfindung wird durch ein Verfahren nach Anspruch 1 gelöst, bei dem die eigentliche Schutzvorrichtung mit Hilfe einer Folie mit der eine Spritze, Nadel oder andere spitze oder scharfe Gegenstände aufweisenden Vorrichtung verbunden wird. Es kann sich bei dieser Folie um ein Kennzeichnungsetikett handeln, das ohnehin zur Kennzeichnung der jeweiligen Vorrichtung notwendig ist, so dass der Etikettierungsprozess gleichzeitig die Ausstattung der Vorrichtung mit dem Schutzmechanismus darstellt.
Die Erfindung schlägt weiter vor, die Schutzvorrichtung selbst aus Folienmaterialien aufzubauen, die in kontinuierlichen Verfahren bearbeitet werden.
Durch die oben beschriebenen Verfahren können derartige Schutzmechanismen preiswert und präzise gefertigt werden. Die Erfindung umfasst daneben auch einen Schutzmechanismus nach Anspruch 9, der mindestens eine Folie enthält sowie eine Anordnung von Schutzmechanismen und eine mit den Schutzmechanismen verbundene Vorrichtung.
Als Folie soll im Folgenden jede Art von rollenförmigen Materialbahnen oder daraus hergestellten Stücken verstanden werden. Neben Kunststoffbahnen fallen darunter auch andere flach aufgerollte Materialien wie beispielsweise Papierbahnen oder dünne Metallfolien. Als Verbindung wird eine Anbringung verstanden, die klebend, vorzugsweise haftklebend ausgeführt ist oder mittels physikalischer oder chemischer Verfahren erfolgt. Unter letztere Kategorie fallen in erster Linie Schweißen, Löten oder Aufschrumpfen, sowohl vollflächig als auch partiell.
Weiterhin soll im Folgenden mit der Bezeichnung "Nadel" der Einfachheit halber jedwede Art von spitzen oder scharfen Gegenständen bezeichnet werden. Hierunter fallen in erster Linie Spritzennadeln, Infusionsnadeln, Akkupunkturnadeln, Kanülen, Lanzetten, Messer und Skalpelle.

Die zahlreichen Ausführungsformen der Erfindung werden im Folgenden anhand der Figuren näher beschrieben. Alle Figuren sind nicht als maßstäblich, sondern schematisch zu verstehen. Speziell die Schichtdicken einzelner Folienlagen sind aus Anschaulichkeitsgründen stark vergrößert dargestellt.

Es zeigen
Fig. 1 einen erfindungsgemäßen Schutzmechanismus, verbunden mit einem Spritzenkörper in seitlicher Ansicht,
Fig. 2 eine weitere Ausführungsform eines erfindungsgemäßen Schutzmechanismus, verbunden mit einem Spritzenkörper in seitlicher Ansicht,
Fig. 3 eine Variante der Ausführungsform aus Figur 2,
Fig. 4 einen erfindungsgemäßen Schutzmechanismus, verbunden mit einem Spritzenkörper und aufgeklappt in seitlicher Ansicht,
Fig. 5 eine spezielle Ausführungsform des erfindungsgemäßen Schutzmechanismus in Draufsicht,
Fig. 6 eine weitere spezielle Ausführungsform des erfindungsgemäßen Schutzmechanismus in seitlicher Schnittansicht,
Fig. 7 eine Ansicht von oben in eine bevorzugte Ausführungsform der Schutzvorrichtung des erfindungsgemäßen Schutzmechanismus,
Fig. 8 eine Vorderansicht einer besonders bevorzugten Ausführungsform des Schutzmechanismus mit Einlassschlitzung,
Fig. 9 a-d verschiedene Formen der Einlasschlitzung gemäß Fig. 8,
Fig. 10 eine Ansicht von oben in eine weitere bevorzugte Ausführungsform der Schutzvorrichtung des erfindungsgemäßen Schutzmechanismus.
Fig. 11 eine Seitenansicht auf eine derartige bevorzugte Ausführungsform der Schutzvorrichtung des erfindungsgemäßen Schutzmechanismus.

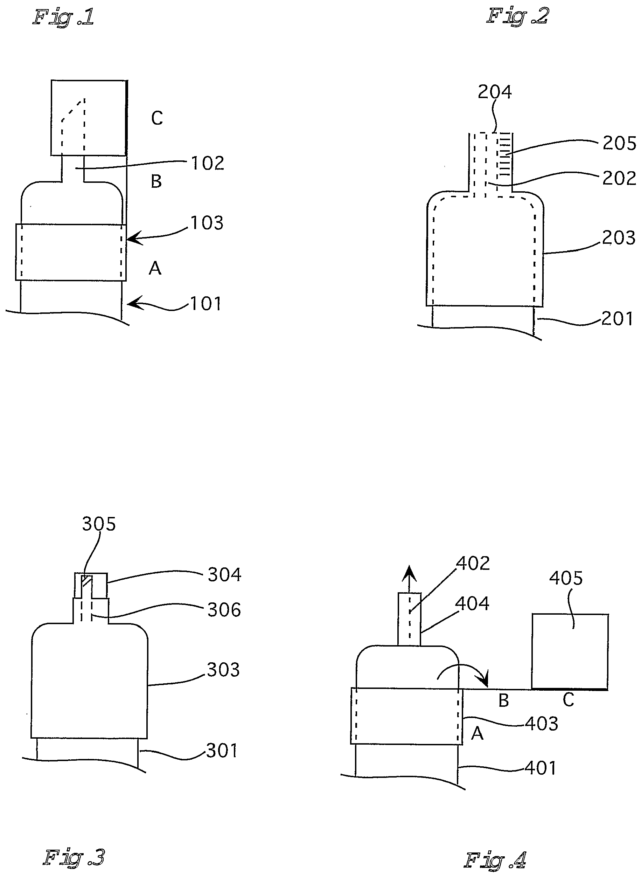

In Fig. 1 ist ein Spritzenkörper 101 dargestellt, der eine Nadel 102 aufweist (in den abgedeckten Bereichen strichliert gezeichnet). Daran ist in einem Bereich A ein erfindungsgemäßer Schutzmechanismus 103 angebracht. Dieser besteht aus drei wesentlichen Bereichen: Dem Verbindungsbereich A, dem Überbrückungsbereich B und dem Bereich der Schutzvorrichtung C. Die Schutzvorrichtung dient dem Schutz von Anwendern der Spritze vor Verletzungen nach Verabreichung der Spritze. Sie kann sowohl als Folie als auch als Hartkunststoffteil oder ähnliches Bauteil aufgebildet sein. Genaue Ausführungsformen der Schutzvorrichtung werden in den Unteransprüchen ausgeführt. Entscheidend für die Erfindung ist, dass mindestens der Bereich A des Schutzmechanismus als Folie ausgebildet ist. Diese Folie dient der Anbringung des gesamten Mechanismus an der Spritze und kann zusätzlich Kennzeichnungszwecke erfüllen. Sie ist in diesem Falle als Etikett ausgeführt. Eine erste bevorzugte Ausführungsform dieser Folie ist, sie als selbstklebende Folie zu gestalten, so dass sie direkt mit der Spritze oder einer ähnlichen Vorrichtung verklebt werden kann. Besonders bevorzugt sind hierbei permanent haftende Klebstoffe, die nicht wasserlöslich sind, um eine Wasserbadbeständigkeit, wie oftmals bei pharmazeutischen Anwendungen gefordert, zu gewährleisten. Als besonders vorteilhafte Ausgangsmaterialien für die Folie haben sich Polyethylentherephtalat (PET) oder Polyolefine (Polyester - PE, Polypropylen - PP oder Polyvinylchlorid - PVC) erwiesen.

Fig. 2 zeigt eine weitere Möglichkeit der Fixierung: Die Bereiche A, B und C aus Fig. 1 sind hier vollumfänglich von einem Folienteil bedeckt, das eine Schutzvorrichtung 205 im Bereich der Nadel 202 aufweist. Diese Figur zeigt die Spritze 201 samt Schutzmechanismus 203 vor Verabreichung einer Spritze, d.h. in ungeöffnetem Zustand. Daher befindet sich um die Nadel 202 eine Schutzkappe 204, die ebenfalls von der Folie des Schutzmechanismus umhüllt ist. Der Schutzmechanismus ummantelt somit den Spritzenkörper fast vollständig bis hin zum oberen Ende der Spritzenkappe. Die Folie des Schutzmechanismus ist daher bevorzugt als sogenannte Schrumpf-Folie schlauchartig ausgebildet. Derartige Schrumpf-Schläuche (Sleeve-Etiketten) können durch Anwendung von Wärme oder durch Recken und Überstülpen auf Gegenstände wie diesen Spritzenkörper aufgebracht werden. Die Folie zieht sich dabei so zusammen, dass sie sich an den ummantelten Körper vollflächig anschmiegt.

Die Schutzvorrichtung 205 besteht in diesem Fall aus einer kamm- oder fühlerartigen Anordnung, die nach Verabreichung der Spritze die offen liegende Nadel umfassen kann. Bevorzugterweise befinden sich an den Enden dieses "Kammes" hin zur Spritze Verdickungen, die ein Einrasten der Nadel ähnlich wie bei einem Klettverschluß ermöglichen.

Ein Vorteil der aufgeschrumpften Ausführungsform der Erfindung ist in Fig. 3 erkennbar. Auf dem Spritzenkörper 301 ist wiederum ein Schutzmechanismus wie 303 in Fig. 2 aufgebracht. In der Zeichnung wurde aus Anschaulichkeitsgründen auf die strichlierte Andeutung des Umrisses des Spritzenkörpers verzichtet. Im Bereich der Nadelschutzkappe 304 weist die (hier nur teilflächig angebrachte) Folie des Schutzmechanismus eine Perforation 306 auf, die es ermöglicht, die Folie in diesem Bereich abzuziehen und somit einen Öffnungsnachweis für die Spritze zu erbringen. Das Aufreissen wird dadurch erleichtert, dass im Bereich der Perforation 306 auch die Schutzvorrichtung 305 unterseitig der Folie angebracht ist. Sie dient als Anfasshilfe zum Öffnen entlang der Perforation. Ähnliche Öffnungsnachweise lassen sich auch bei selbstklebenden Ausführungsformen des Schutzmechanismus realisieren. In diesem Fall würde die Nadelschutzkappe von einer auf sich selbst klebenden Folie ummantelt, die entweder ebenfalls eine Aufreißperforation oder beispielsweise einen Öffnungsnachweis in Form des sogenannten VOID-Effekts aufweist. Dieser bewirkt die Ausbildung eines irreversibel generierten Schriftzugs oder Zeichens aus Druckfarbe im geöffneten Bereich.

Fig. 4 zeigt in seitlicher Ansicht einen erfindungsgemäßen Schutzmechanismus auf einem Spritzenkörper in aufgeklappten Zustand: Dargestellt ist der Spritzenkörper 403, der eine Nadelschutzkappe 404 aufweist, die eine Nadel 402 (strichliert angedeutet) vor der Verarbreichung der Spritze schützt. Der Schutzmechanismus 403 mit der Schutzvorrichtung 405 ist im 90 Grad-Winkel von der Schutzkappe weggeklappt, so dass diese nun abgenommen und die Spritze verabreicht werden kann. Nach der Verabreichung wird der Schutzmechanismus wieder zurückgeklappt, so dass die Schutzvorrichtung die Nadel aufnimmt und den Nutzer vor Verletzungen schützt. Um das Verabreichen der Spritze ohne Behinderungen für den Nutzer zu gewährleisten ist es notwendig, dass der Mechanismus im Übergangsbereich zwischen A und B einfach und definiert umklappt. Um dies zu gewährleisten, können in diesem Übergangsbereich verschiedene Maßnahmen ergriffen werden.

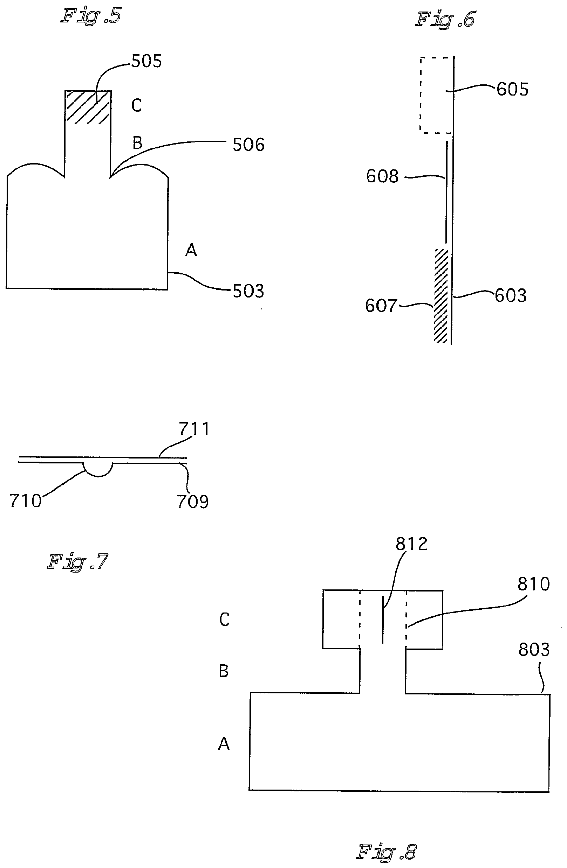

Fig. 5 zeigt einen Schutzmechanismus 503 mit einer nicht näher dargestellten durch Schraffur gekennzeichneten Schutzvorrichtung für Nadeln, bei dem im Übergangsbereich zwischen A und B eine Stanzung 506 vorgenommen wurde. Durch diese Stanzung klappt bei Aufbringung des Schutzmechanismus auf eine zylindrische oder zylinder-ähnliche Vorrichtung der Bereich B aufgrund der durch die Zylinderform vorgegebenen Biegung des Übergangsbereiches definiert um.
Ein ähnlicher Effekt ist erreichbar durch den Aufbau in Fig. 6, in der ein Schutzmechanismus 603 mit einer nicht näher dargestellten Schutzvorrichtung 605 und eine Klebstoffschicht 607 (zur Fixierung am Objekt) dargestellt ist. Im Überbrückungsbereich B ist eine zusätzliche Folie 608 angebracht, die diesen Bereich verstärkt und somit zu einer Schwächung der Folie im Übergangsbereich zwischen A und B führt. Statt einer zusätzlichen Folie ist es auch möglich, einen nach unten auslaufenden Teil der (beispielsweise spritzgegossenen) Schutzvorrichtung als Verstärkung zu verwenden. Eine Schwächung dieses Übergangsbereichs ist auch dadurch zu erreichen, dass dort eine Prägung oder eine Verringerung der Breite des Überbrückungsbereichs vorgenommen wird.
Generell wird der Effekt eines definierten Umklappens durch erhöhte Materialsteifigkeit im Überbrückungsbereich B und/oder Schwächung im Übergangsbereich zwischen Überbrückungs- und Verbindungsbereich erreicht. Vorzugsweise werden beide Maßnahmen in geeigneter Kombination angewandt, um im Zusammenspiel eine genau definierbare Umklapp-Wirkung zu erzielen.
In den folgenden Figuren wird nun auf besonders bevorzugte Ausführungsformen der eigentlichen Schutzvorrichtung des Schutzmechanismus eingegangen. Prinzipiell kann die Schutzvorrichtung ebenfalls aus mindestens einer Folie gebildet sein oder aus einem anderweitigen, üblicherweise aus Kunststoff hergestellten Teil, bestehen.
Fig. 7 zeigt eine besonders bevorzugte Variante eines Folienteils in der Ansicht von oben. Eine erste Folie 709 ist in einem Bereich 710 so verformt, dass in die Ausbuchtung eine Nadel eingebracht werden kann. Um diesen Gegenstand von allen Seiten zu umfassen, ist üblicherweise auf der Rückseite der Folie eine zweite Folie 711 aufgebracht. In den Hohlraum dazwischen wird - gewöhnlich durch eine Schlitzung in einer der beiden Folien 709 oder 711 - die Nadel oder ein anderer zu schützender Gegenstand eingeführt. Eine spezielle Form dieses Folienteils besteht darin, dass die Ausbuchtung im Bereich 710 als eine doppelte Buchtung ausgeführt ist. Andere Formen der Buchtung sind ebenso möglich und korrespondieren vorteilhafterweise mit der Form des zu schützenden Gegenstands.
Fig. 8 zeigt einen entsprechenden Schutzmechanismus 803 in der Vorderansicht. Der Bereich der Ausbuchtung 810 ist hier strichliert dargestellt. Zu erkennen ist in dieser Figur auch die Schlitzung, in die die Nadel eingeführt wird.

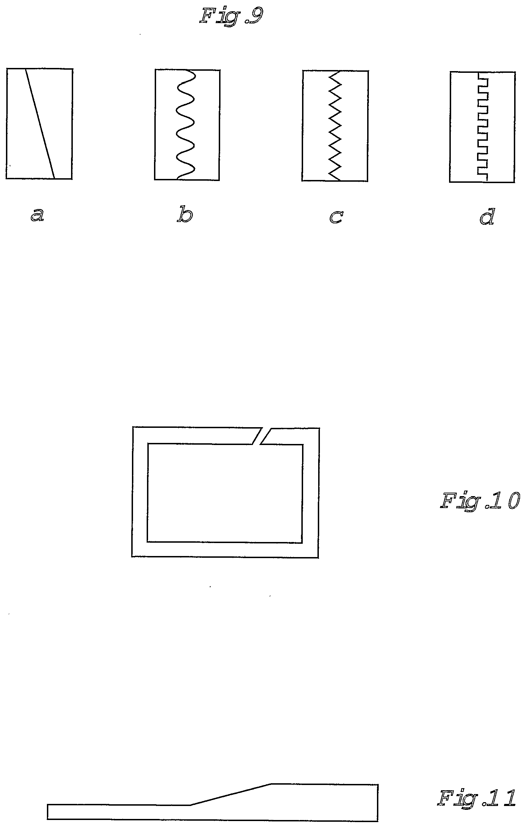

Es kann sich als sinnvoll erweisen, die Schlitzung nicht in gerader Form durchzuführen, wie in Figuren 9 a-d dargestellt. 9a zeigt eine schräg verlaufende Schlitzung, die nicht senkrecht zur Kante der Folie verläuft, 9b einen Wellenverlauf, 9c eine Zickzacklinie und 9d einen zinnenartigen Verlauf. Hierdurch kann gewährleistet werden, dass der zu schützende Gegenstand zwar mit etwas größerem Aufwand in den Hohlraum hinter der Schlitzung einfährt, jedoch noch schwieriger wieder entweichen kann. Speziell gilt dies dann, wenn die Folie zusätzlich nach dem Einbringen der Schlitzung verformt (beispielsweise geschrumpft oder in sich verdreht) wird und dadurch ein Überstand der durch die Schlitzung hergestellten Folienbereiche entsteht.

Wie angedeutet können alternativ zu den vorgestellten Schutzvorrichtungen, die aus einer oder mehreren Folien bestehen, auch nicht-folienartige Teile verwendet werden. In erster Linie handelt es sich dabei um spritzgegossene oder aus Strangprofilen hergestellte Teile. Eine mögliche Form eines solchen Formteils ist in Fig. 10 in Ansicht von oben dargestellt. Auch hier wird wieder - analog zur Schlitzung in den vorherigen Beispielen - ein Einlaß für die Nadel bereitgestellt. Zahlreiche Formen sind für derartige Schutzvorrichtungen vorstellbar, die sich auch an den bekannten Stand der Technik anlehnen können. Die Aufnahme von Flüssigkeiten, speziell potenziell infizierter Körperflüssigkeiten, kann durch die Verwendung feuchtigkeitsabsorbierender Materialien wie Geweben oder Vliesstoffen, zusätzlich gewährleistet werden. Als geeignete Materialen für spritzgegossene oder aus Strangprofilen hergestellte Teile haben sich in erster Linie Polystyrol (PS), AcrylnitrilButadien-Styrol (ABS), Polycarbonat (PC) und thermoplastisches Urethan (TPU) herausgestellt.
In Fig. 11 ist eine derartige Schutzvorrichtung in Seitenansicht dargestellt. In dem Bereich - hier rechts dargestellt -, in dem die Nadel aufgenommen werden soll, ist das Profil größer ausgebildet, um genügend Raum für die Nadel bereitzustellen. In dem Bereich, der in erster Linie im Überbrückungsbereich B zum Liegen kommt und der mit der Folie des Schutzmechanismus verbunden ist, kann die Dicke der Schutzvorrichtung deutlich geringer sein. Sie kann - wie oben beschrieben - zur Verstärkung der Folie und damit zu einer Gewährleistung eines definierten Umklappmechanismus dienen.

Die übliche Ausformung der vorliegenden Erfindung besteht darin, mindestens einen Teil des Folienbereichs als Etikett, d.h. als bedruckte Folie bereitzustellen, da hierdurch ein Prozessschritt bei der Bereitstellung von Spritzen oder anderen Vorrichtungen gespart werden kann: Die Applikation des Schutzmechanismus ist gleichzeitig die Etikettierung. Die Bedruckung kann sich insbesondere auf die Benutzung des Schutzmechanismus beziehen. Neben der reinen Kennzeichnungsfunktion können Etiketten weitere Funktionsmerkmale enthalten, die ebenfalls bei der hier vorliegenden Erfindung zur Anwendung kommen können: Beispielsweise ist es möglich, das Etikett mit abnehmbaren selbstklebenden Belegteilen auszustatten, die beispielsweise nach Verabreichung einer Spritze als Nachweis in eine Krankenakte verklebt werden. Ebenso ist die Integration von folienartigen Aufhängevorrichtungen möglich oder ein Bereich, dessen Oberfläche so behandelt ist, dass er mittels Nachbeschriftungsverfahren auch nach der Bereitstellung des Schutzmechanismus durch den Hersteller bedruckbar ist.
Erfindungsgemäße Schutzmechanismen wie hier beschrieben werden gewöhnlich in automatisierten Spende- oder Aufbringungsverfahren mit den vorgesehenen Vorrichtungen verbunden. Hierzu ist es besonders sinnvoll und kostenmindernd, die Mechanismen im Endlos-Verfahren bereitzustellen und zu applizieren. Erfindungsgemäß werden daher die Schutzmechanismen unmittelbar oder mittelbar miteinander verbunden. Dies erfolgt bei den schlauchartigen Ausführungsformen (Sleeve-Technologie) üblicherweise dadurch, dass die einzelnen Schläuche endlos hintereinandergereiht, eventuell separiert durch Perforationen oder andere Schwächungen, angeliefert werden und erst beim Aufbringen des Schlauches auf die Vorrichtungen voll voneinander getrennt, beispielsweise abgeschnitten oder an den Schwächungslinien getrennt werden. Bei selbstklebenden Lösungen hingegen bietet es sich an, die Schutzmechanismen auf einer Trägerbahn anzuordnen, wobei es aus Herstellungs- und Anordnungsgründen besonders sinnvoll ist, den Bereich der Schutzvorrichtung senkrecht zur Bahnlaufrichtung anzuordnen. Der Bereich der Schutzvorrichtung steht dabei üblicherweise weiter vom Träger ab als die restlichen Bereiche, weshalb hier zur Einsparung von Höhe eine Aussparung der Trägerbahn vorgesehen werden kann. Hierdurch lassen sich die Schutzmechanismen auf dem Trägerband einfacher auf Rolle aufrollen. Eine besondere Form der Aufrollung besteht darin, die Schutzmechanismen auf ihrer Trägerbahn in einer Spendekassette, einem Dispenser bereitzustellen. Hierbei werden die Schutzmechanismen in der Kassette und speziell beim Verspenden so gehalten, dass sie trotz der Dickenunterschiede aufgrund des Schutzmechanismus komplikationslos aufgerollt und verspendbar sind. Weiterhin ist es besonders vorteilhaft, ein besonders dickes Trägerpapier zu verwenden, um eine Wechselwirkung der verschiedenen übereinanderliegenden Lagen von Schutzmechanismen im aufgerollten Zustand zu vermeiden.

## Patentansprüche

1. Verfahren zur Herstellung einer Vorrichtung mit einem Schutzmechanismus, umfassend:
- Bereitstellen eines Körpers (101) mit einer verletzungsgefährlichen Nadel (102);
- Bereitstellen mindestens einer Folie;
- Bereitstellen einer Schutzvorrichtung (C), die als Hartkunststoffteil ausgebildet ist oder als eine erste Folie (709) und eine auf der Rückseite der ersten Folie (709) aufgebrachte zweite Folie (711) mit einer in der ersten oder der zweiten Folie (709, 711) eingebrachten Schlitzung ausgeführt ist, wobei die erste Folie (709) zu einer Ausbuchtung verformt ist, in die eine Nadel eingebracht werden kann;
- Verbinden der mindestens einen Folie mit der Schutzvorrichtung (C) unter Vorsehen eines Überbrückungsbereichs (B), der zwischen der Folie und der Schutzvorrichtung (C) angeordnet ist, wobei weiterhin Mittel vorgesehen sind, die ein Umklappen des Überbrückungsbereichs (B) im Übergangsbereich zwischen einem Verbindungsbereich (A) der mindestens einen Folie und dem Überbrückungsbereich (B) ermöglichen;
- Befestigen der mindestens einen Folie im Verbindungsbereich (A) an dem Körper (101).

2. Verfahren nach Anspruch 1 , **dadurch gekennzeichnet, dass** der Schutzmechanismus aus einem bahnförmigen Ausgangsmaterial hergestellt wird.

3. Verfahren nach Anspruch 2, **dadurch gekennzeichnet, dass** das bahnförmige Ausgangsmaterial nach der Fertigung auf eine Rolle gewickelt wird.

4. Verfahren nach einem der Ansprüche 1 bis 3, **dadurch gekennzeichnet, dass** der Schutzmechanismus in einem kontinuierlichen Spendeverfahren auf die Vorrichtung aufgebracht wird.

5. Verfahren nach einem der Ansprüche 1 bis 4, **dadurch gekennzeichnet, dass** das Befestigen der mindestens einen Folie an dem Körper (101) mit Hilfe eines Klebstoffs durchgeführt wird.

6. Verfahren nach einem der Ansprüche 1 bis 5, **dadurch gekennzeichnet, dass** das Befestigen der mindestens einen Folie an dem Körper (101) mit Hilfe eines Haftklebstoffs durchgeführt wird.

7. Verfahren nach einem der Ansprüche 1 bis 5, **dadurch gekennzeichnet, dass** das Befestigen der mindestens einen Folie an dem Körper (101) mit Hilfe eines permanent haftenden Klebstoffs durchgeführt wird.

8. Verfahren nach einem der Ansprüche 1 bis 4, **dadurch gekennzeichnet, dass** die mindestens eine Folie zur Befestigung an dem Körper (101) auf diesen geschrumpft wird.

9. Schutzmechanismus für einen Körper mit einer verletzungsgefährlichen Nadel, umfassend:
- einen Verbindungsbereich (A), der als mindestens eine Folie ausgebildet ist und an dem Körper (101) befestigt ist;
- eine zum Schutz vor Verletzung und zur Aufnahme der Nadel (102) dienende Schutzvorrichtung (C), die als Hartkunststoffteil ausgebildet ist oder als eine erste Folie (709) und eine auf der Rückseite der ersten Folie aufgebrachte zweite Folie (711) mit einer in der ersten oder der zweiten Folie eingebrachten Schlitzung ausgeführt ist, wobei die erste Folie (709) zu einer Ausbuchtung verformt ist, in die eine Nadel eingebracht werden kann;
- einen Überbrückungsbereich (B), der zwischen dem Verbindungsbereich (A) und der Schutzvorrichtung (C) angeordnet ist; **gekennzeichnet durch** Mittel, die ein Umklappen des Überbrückungsbereichs (B) im Übergangsbereich zwischen dem Verbindungsbereich (A) und dem Überbrückungsbereich (B) ermöglichen.

10. Schutzmechanismus nach Anspruch 9, **dadurch gekennzeichnet, dass** die mindestens eine Folie aus PET, PE, PP oder PVC hergestellt ist.

11. Schutzmechanismus nach Anspruch 9 oder 10, **dadurch gekennzeichnet, dass** die mindestens eine Folie Mittel zur Anbringung auf dem Körper (101) aufweist.

12. Schutzmechanismus nach Anspruch 11 , **dadurch gekennzeichnet, dass** die mindestens eine Folie auf dem Körper (101) mindestens teilweise aufgeschrumpft ist.

13. Schutzmechanismus nach Anspruch 12, **dadurch gekennzeichnet, dass** die mindestens eine Folie durch Einwirkung von Wärme aufgeschrumpft ist.

14. Schutzmechanismus nach Anspruch 12, **dadurch gekennzeichnet, dass** die mindestens eine Folie durch chemische oder durch Wärme oder Strahlung aufgeschrumpft ist.

15. Schutzmechanismus nach Anspruch 11, **dadurch gekennzeichnet, dass** die mindestens eine Folie reckbar ist und schlauchförmig auf den Körper (101) unter gleichzeitigem Zusammenziehen aufgestülpt ist.

16. Schutzmechanismus nach einem der Ansprüche 12 bis 15, **dadurch gekennzeichnet, dass** die mindestens eine Folie Mittel aufweist, die im auf den Körper (101) angebrachten Zustand ein Aufreissen ermöglichen.

17. Schutzmechanismus nach Anspruch 16, **dadurch gekennzeichnet, dass** es sich bei den Mitteln zum Aufreissen um eine Perforation handelt.

18. Schutzmechanismus nach Anspruch 16, **dadurch gekennzeichnet, dass** es sich bei den Mitteln zum Aufreissen um eine Prägung handelt.

19. Schutzmechanismus nach Anspruch 16, **dadurch gekennzeichnet, dass** es sich bei den Mitteln zum Aufreissen um eine Anstanzung handelt.

20. Schutzmechanismus nach einem der Ansprüche 16 bis 19, **dadurch gekennzeichnet, dass** die Mittel zum Aufreissen in dem Bereich liegen, in dem die mindestens eine Folie im auf den Körper (101) angebrachten Zustand im Bereich der verletzungsgefährlichen Nadel zum Liegen kommt.

21. Schutzmechanismus nach Anspruch 20, **dadurch gekennzeichnet, dass** die Mittel zum Aufreissen das Abnehmen eines Folienteils in dem Bereich ermöglichen, in dem die mindestens eine Folie im auf den Körper (101) angebrachten Zustand im Bereich der verletzungsgefährlichen Nadel zum Liegen kommt.

22. Schutzmechanismus nach Anspruch 11, **dadurch gekennzeichnet, dass** als Mittel zur Anbringung auf den Körper (101) eine Klebstoffschicht dient.

23. Schutzmechanismus nach Anspruch 22, **dadurch gekennzeichnet, dass** der Klebstoff wasserunlöslich ist.

24. Schutzmechanismus nach Anspruch 22 oder 23, **dadurch gekennzeichnet, dass** es sich bei dem Klebstoff um einen permanent haftenden Klebstoff handelt.

25. Schutzmechanismus nach einem der Ansprüche 22 bis 24, **dadurch gekennzeichnet, dass** die volle Klebewirkung des Klebstoff nur teilflächig vorliegt.

26. Schutzmechanismus nach Anspruch 25, **dadurch gekennzeichnet, dass** der Klebstoff nur teilflächig aufgebracht ist.

27. Schutzmechanismus nach Anspruch 25, **dadurch gekennzeichnet, dass** der Klebstoff teilflächig mit Mitteln zur Abschwächung oder Aufhebung seiner Klebewirkung behandelt ist.

28. Schutzmechanismus nach einem der Ansprüche 9 bis 27, **dadurch gekennzeichnet, dass** die Folie mindestens ein weiteres Funktionsteil aufweist.

29. Schutzmechanismus nach Anspruch 28, **dadurch gekennzeichnet, dass** es sich bei dem mindestens einen weiteren Funktionsteil um mindestens einen abnehmbaren Beleg handelt.

30. Schutzmechanismus nach Anspruch 28, **dadurch gekennzeichnet, dass** es sich bei dem mindestens einen weiteren Funktionsteil um mindestens einen Aufhängevorrichtung handelt.

31. Schutzmechanismus nach einem der Ansprüche 9 bis 30, **dadurch gekennzeichnet, dass** die mindestens eine Folie eine Bedruckung aufweist.

32. Schutzmechanismus nach Anspruch 31, **dadurch gekennzeichnet, dass** es sich bei der Bedruckung um Informationen zur Benutzung des Schutzmechanismus handelt.

33. Schutzmechanismus nach einem der Ansprüche 9 bis 32, **dadurch gekennzeichnet, dass** die mindestens eine Folie einen nachbedruckbaren Bereich ausweist.

34. Schutzmechanismus nach Anspruch 33, **dadurch gekennzeichnet, dass** der Verbindungsbereich (A) und der Überbrückungsbereich (B) aus der selben Folie ausgebildet sind.

35. Schutzmechanismus nach Anspruch 34, **dadurch gekennzeichnet, dass** der Verbindungsbereich (A) und der Überbrückungsbereich (B) sowie die Schutzvorrichtung (C) aus der selben Folie ausgebildet sind.

36. Schutzmechanismus nach Anspruch 33 oder 34, **dadurch gekennzeichnet, dass** es sich bei der Schutzvorrichtung (C) um ein an mindestens dem Überbrückungsbereich (B) befestigtes Funktionsteil handelt.

37. Schutzmechanismus nach Anspruch 9, **dadurch gekennzeichnet, dass** es sich bei der ersten oder der zweiten Folie (709, 711) um eine verformte Folie handelt.

38. Schutzmechanismus nach Anspruch 37, **dadurch gekennzeichnet, dass** es sich bei der ersten oder der zweiten Folie (709, 711) um eine tiefgezogene Folie handelt.

39. Schutzmechanismus nach Anspruch 38, **dadurch gekennzeichnet, dass** die tiefgezogene Folie von der jeweils anderen der ersten und der zweiten Folien (711, 709) mindestens teilflächig abgedeckt ist.

40. Schutzmechanismus nach Anspruch 38 oder 39, **dadurch gekennzeichnet, dass** die tiefgezogene Folie die Schlitzung aufweist.

41. Schutzmechanismus nach Anspruch 40, **dadurch gekennzeichnet, dass** die Schlitzung nicht senkrecht zu einer der Kanten der jeweiligen Folie(n) endet.

42. Schutzmechanismus nach Anspruch 41, **dadurch gekennzeichnet, dass** die Schlitzung wellenförmig verläuft.

43. Schutzmechanismus nach Anspruch 41, **dadurch gekennzeichnet, dass** die Schlitzung in Zickzack- oder Zinnenform verläuft.

44. Schutzmechanismus nach einem der Ansprüche 38 bis 43, **dadurch gekennzeichnet, dass** die tiefgezogene Folie geschrumpft ist.

45. Schutzmechanismus nach einem der Ansprüche 38 bis 44, **dadurch gekennzeichnet, dass** sich die durch die Schlitzung erzeugten Folienbereiche nach der Schlitzung überlappen.

46. Schutzmechanismus nach einem der Ansprüche 33 bis 45, **dadurch gekennzeichnet, dass** der Verbindungsbereich (A) und/oder der Überbrückungsbereich (B) die Mittel aufweisen, die ein definiertes Umklappen des Überbrückungsbereichs (B) im auf den Körper (101) aufgebrachten Zustand ermöglichen.

47. Schutzmechanismus nach Anspruch 46, **dadurch gekennzeichnet, dass** es sich bei den Mitteln zur Ermöglichung des definierten Umklappens um eine Dickenverstärkung mindestens des Überbrückungsbereichs (B) handelt.

48. Schutzmechanismus nach Anspruch 47, **dadurch gekennzeichnet, dass** die Dickenverstärkung aus mindestens einer weiteren Folienlage besteht.

49. Schutzmechanismus nach Anspruch 47 oder 48, **dadurch gekennzeichnet, dass** die Dickenverstärkung aus Teilen der Schutzvorrichtung (C) gebildet ist.

50. Schutzmechanismus nach Anspruch 46, **dadurch gekennzeichnet, dass** es sich bei den Mitteln zur Ermöglichung des definierten Umklappens um eine Schwächung am Übergang zwischen Verbindungsbereich (A) und Überbrückungsbereich (B) handelt.

51. Schutzmechanismus nach Anspruch 50, **dadurch gekennzeichnet, dass** die Schwächung am Übergang zwischen dem Verbindungsbereich (A) und dem Überbrückungsbereich (B) eine Stanzung oder Schlitzung ist.

52. Schutzmechanismus nach Anspruch 50, **dadurch gekennzeichnet, dass** die Schwächung am Übergang zwischen dem Verbindungsbereich (A) und dem Überbrückungsbereich (B) eine Prägung ist.

53. Schutzmechanismus nach Anspruch 50, **dadurch gekennzeichnet, dass** die Schwächung am Übergang zwischen dem Verbindungsbereich (A) und dem Überbrückungsbereich (B) eine Verringerung der Breite des Überbrückungsbereichs ist.

54. Schutzmechanismus nach einem der Ansprüche 46 bis 53, **dadurch gekennzeichnet, dass** sowohl eine Dickenverstärkung als auch eine Schwächung am Übergang zwischen dem Verbindungsbereich (A) und dem Überbrückungsbereich (B) vorliegt und diese in ihrer Wirkung so aufeinander abgestimmt sind, dass ein definiertes Umklappen ermöglicht wird.

55. Schutzmechanismus nach Anspruch 36, **dadurch gekennzeichnet, dass** es sich bei dem Funktionsteil um ein Spritzgussteil handelt.

56. Schutzmechanismus nach Anspruch 36, **dadurch gekennzeichnet, dass** es sich bei dem Funktionsteil um ein aus einem Strangprofil hergestelltes Teil handelt.

57. Schutzmechanismus nach Anspruch 55 oder 56, **dadurch gekennzeichnet, dass** es sich bei dem Funktionsteil um ein Teil aus PS, PP, ABS oder TPU handelt.

58. Schutzmechanismus nach Anspruch 36, **dadurch gekennzeichnet, dass** es sich bei dem Funktionsteil um ein kamm- oder fühlerartiges Bauteil handelt.

59. Schutzmechanismus nach einem der Ansprüche 9 bis 58, **dadurch gekennzeichnet, dass** es ein feuchtigkeitsabsorbierendes Material aufweist.

60. Anordnung von Schutzmechanismen nach einem der Ansprüche 9 bis 59, bei der die Schutzmechanismen mittelbar oder unmittelbar miteinander verbunden sind.

61. Anordnung nach Anspruch 60, **dadurch gekennzeichnet, dass** die Schutzmechanismen in Form eines Schlauches hintereinander angeordnet sind.

62. Anordnung nach Anspruch 61, **dadurch gekennzeichnet, dass** die einzelnen Schutzmechanismen durch Schwächungen des Schlauches voneinander getrennt sind.

63. Anordnung nach Anspruch 62, **dadurch gekennzeichnet, dass** es sich bei den Schwächungen um Perforationsstanzungen handelt.

64. Anordnung nach Anspruch 60, **dadurch gekennzeichnet, dass** die Schutzmechanismen klebend auf einer Trägerbahn angeordnet sind.

65. Anordnung nach Anspruch 64, **dadurch gekennzeichnet, dass** die Schutzvorrichtung (C) der Schutzmechanismen senkrecht zur Bahnlaufrichtung zum Liegen kommt.

66. Anordnung nach Anspruch 65, **dadurch gekennzeichnet, dass** im Bereich der Schutzvorrichtungen (C) eine Aussparung der Trägerbahn vorgesehen ist.

67. Eine verletzungsgefährliche Nadel enthaltende Vorrichtung, mit einem Schutzmechanismus nach einem der Ansprüche 9 bis 59.

68. Vorrichtung nach Anspruch 67, **dadurch gekennzeichnet, dass** der Schutzmechanismus selbstklebend an der Vorrichtung angebracht ist.

69. Vorrichtung nach Anspruch 67, **dadurch gekennzeichnet, dass** der Schutzmechanismus um die Vorrichtung umlaufend angebracht ist.

70. Vorrichtung nach Anspruch 69, **dadurch gekennzeichnet, dass** der Schutzmechanismus auf die Vorrichtung aufgeschrumpft ist.

## Claims

1. A method of manufacturing a device having a protective mechanism, comprising:
- providing a body (101) having an injury-prone needle (102),
- providing at least one film;
- providing a protective device (C) which is formed as a rigid plastic part or designed as a first film (709) and a second film (711) applied to the back of the first film (709) with a slit worked into the first or second film (709, 711), wherein the first film (709) is deformed to result in a bulge into which a needle can be inserted;
- connecting the at least one film to the protective device (C) by providing a bridging area (B) disposed between the film and the protective device (C), means being further provided to allow the bridging area (B) to fold over in the transition area between a connection area (A) of the at least one film and the bridging area (B);
- fastening the at least one film in the connection area (A) to the body (101).

2. The method according to claim 1, **characterized in that** the protective mechanism is manufactured from a web-shaped base material.

3. The method according to claim 2, **characterized in that** the web-shaped base material is wound onto a reel after manufacture.

4. The method according to any of claims 1 to 3, **characterized in that** the protective mechanism is applied to the device in a continuous dispensing method.

5. The method according to any of claims 1 to 4, **characterized in that** the process of fastening the at least one film to the body (101) is carried out with the aid of an adhesive.

6. The method according to any of claims 1 to 5, **characterized in that** the process of fastening the at least one film to the body (101) is carried out with the aid of a pressure-sensitive adhesive.

7. The method according to any of claims 1 to 5, **characterized in that** the process of fastening the at least one film to the body (101) is carried out with the aid of a permanent adhesive.

8. The method according to any of claims 1 to 4, **characterized in that** the at least one film for fastening to the body (101) is applied thereon by shrinking.

9. A protective mechanism for a body having an injury-prone needle, comprising:
- a connecting region (A) formed as at least one film and fastened to the body (101);
- a protective device (C) for protection against injury and for receiving the needle (102), said protective device being formed as a rigid plastic part or designed as a first film (709) and a second film (711) applied to the back of the first film (709) with a slit worked into the first or second film (709, 711), wherein the first film (709) is deformed to result in a bulge into which a needle can be inserted;
- a bridging area (B) disposed between the connection area (A) and the protective device (C);
**characterized by**
means enabling the bridging area (B) to fold over in the transition area between the connection area (A) and the bridging area (B).

10. The protective mechanism according to claim 9, **characterized in that** the at least one film is made of PET, PE, PP or PVC.

11. The protective mechanism according to claim 9 or 10, **characterized in that** the at least one film comprises means for attaching to the body (101).

12. The protective mechanism according to claim 11, **characterized in that** the at least one film is at least partially applied on the body (101) by shrinking.

13. The protective mechanism according to claim 12, **characterized in that** the at least one film is applied by shrinking under the action of heat.

14. The protective mechanism according to claim 12, **characterized in that** the at least one film is applied by shrinking under the action of chemical agents or heat or radiation.

15. The protective mechanism according to claim 11, **characterized in that** the at least one film is stretchable and is put over the body (101) in the form of a tube with simultaneous contraction.

16. The protective mechanism according to any of claims 12 to 15, **characterized in that** the at least one film comprises means which, when applied to the body (101), allow tearing.

17. The protective mechanism according to claim 16, **characterized in that** the means for tearing represent a perforation.

18. The protective mechanism according to claim 16, **characterized in that** the means for tearing represent an embossing.

19. The protective mechanism according to claim 16, **characterized in that** the means for tearing represent a partially punching.

20. The protective mechanism according to any of claims 16 to 19, **characterized in that** the means for tearing are located in the area in which the at least one film comes to rest in the area of the injury-prone needle when attached to the body (101).

21. The protective mechanism according to claim 20, **characterized in that** the means for tearing comprise the removal of a film section in the area in which the at least one film comes to rest in the area of the injury-prone needle when attached to the body (101).

22. The protective mechanism according to claim 11, **characterized in that** the means for attaching to the body (101) are represented by an adhesive layer.

23. The protective mechanism according to claim 22, **characterized in that** the adhesive is insoluble in water.

24. The protective mechanism according to claim 22 or 23, **characterized in that** the adhesive is a permanent adhesive.

25. The protective mechanism according to any of claims 22 to 24, **characterized in that** the full adhesive effect of the adhesive is only present in parts.

26. The protective mechanism according to claim 25, **characterized in that** the adhesive is applied only in parts.

27. The protective mechanism according to claim 25, **characterized in that** the adhesive is treated in parts with agents for weakening or canceling its adhesive effect.

28. The protective mechanism according to any of claims 9 to 27, **characterized in that** the film has at least one further functional part.

29. The protective mechanism according to claim 28, **characterized in that** the at least one further functional part is at least one removable document.

30. The protective mechanism according to claim 28, **characterized in that** the at least one further functional part is at least one suspension device.

31. The protective mechanism according to any of claims 9 to 30, **characterized in that** the at least one film has a printing on it.

32. The protective mechanism according to claim 31, **characterized in that** the printing is information on the use of the protective mechanism.

33. The protective mechanism according to any of claims 9 to 32, **characterized in that** the at least one film comprises a zone which can be imprinted at a later point in time.

34. The protective mechanism according to claim 33, **characterized in that** the connection region (A) and the bridging region (B) are formed from the same film.

35. The protective mechanism according to claim 34, **characterized in that** the connection region (A) and the bridging region (B) as well as the protective device (C) are formed from the same film.

36. The protective mechanism according to claim 33 or 34, **characterized in that** the protective device (C) is a functional part which is fixed to at least the bridging region (B).

37. The protective mechanism according to claim 9, **characterized in that** the first or second film (709, 711) is a deformed film.

38. The protective mechanism according to claim 37, **characterized in that** the first or second film (709, 711) is a deep-drawn film.

39. The protective mechanism according to claim 38, **characterized in that** the deep-drawn film is covered at least in parts by the respective other of the first and second films (711, 709).

40. The protective mechanism according to claim 38 or 39, **characterized in that** the deep-drawn film is provided with the slit.

41. The protective mechanism according to claim 40, **characterized in that** the slit does not end perpendicular to one of the edges of the respective film(s).

42. The protective mechanism according to claim 41, **characterized in that** the slit extends in a wavy shape.

43. The protective mechanism according to claim 41, **characterized in that** the slit extends in a zigzag or crenelated shape.

44. The protective mechanism according to any of claims 38 to 43, **characterized in that** the deep-drawn film is a shrink-type film.

45. The protective mechanism according to any of claims 38 to 44, **characterized in that** the film regions produced by the slitting overlap after the slitting.

46. The protective mechanism according to any of the claims 33 to 45, **characterized in that** the connection region (A) and/or the bridging region (B) have means enabling a defined folding of the bridging region (B) in the state when applied to the body (101).

47. The protective mechanism according to claim 46, **characterized in that** the means for enabling the defined folding are realized in the form of thickened zone of at least the bridging region (B).

48. The protective mechanism according to claim 47, **characterized in that** the thickened zone is formed from at least one further film layer.

49. The protective mechanism according to claim 47 or 48, **characterized in that** the thickened zone is formed from parts of the protective device (C).

50. The protective mechanism according to claim 46, **characterized in that** the means for enabling the defined folding is a weakening zone at the transition between the connection region (A) and the bridging region (B).

51. The protective mechanism according to claim 50, **characterized in that** the weakening zone at the transition between the connection region (A) and the bridging region (B) is a punching or slitting.

52. The protective mechanism according to claim 50, **characterized in that** the weakening zone at the transition between the connection region (A) and the bridging region (B) is an embossing.

53. The protective mechanism according to claim 50, **characterized in that** the weakening zone at the transition between the connection region (A) and the bridging region (B) is realized by a reduced width of the bridging region.

54. The protective mechanism according to any of claims 46 to 53, **characterized in that** there is both a thickened zone and a weakening zone at the transition between the connection region (A) and the bridging region (B) and their effect is coordinated in such a way that defined folding is made possible.

55. The protective mechanism according to claim 36, **characterized in that** the functional part is an injection-molded part.

56. The protective mechanism according to claim 36, **characterized in that** the functional part is a part made of an extruded profile.

57. The protective mechanism according to claim 55 or 56, **characterized in that** the functional part is a part made of PE, PP, ABS or TPU.

58. The protective mechanism according to claim 36, **characterized in that** the functional part is a comb- or tentacle-like component.

59. The protective mechanism according to any of claims 9 to 58, **characterized in that** it comprises a moisture-absorbing material.

60. An assembly of protective mechanisms according to any of claims 9 to 59, in which the protective mechanisms are directly or indirectly connected to one another.

61. The assembly according to claim 60, **characterized in that** the protective mechanisms are arranged one behind the other in the form of a hose.

62. The assembly according to claim 61, **characterized in that** the individual protective mechanisms are separated from one another by weakening zones of the hose.

63. The assembly according to claim 62, **characterized in that** the weakening zones are realized by perforation-type punching.

64. The assembly according to claim 60, **characterized in that** the protective mechanisms are adhesively arranged on a carrier web.

65. The assembly according to claim 64, **characterized in that** the protective device (C) of the protective mechanisms comes to rest perpendicularly to the web's running direction.

66. The assembly according to claim 65, **characterized in that** a recess in the carrier web is provided in the region of the protective devices (C).

67. A device with an injury-prone needle, comprising a protective mechanism according to any of claims 9 to 59.

68. The device according to claim 67, **characterized in that** the protective mechanism is self-adhesively attached to the device.

69. The device according to claim 67, **characterized in that** the protective mechanism is attached circumferentially around the device.

70. The device according to claim 69, **characterized in that** the protective mechanism is applied onto the device by shrinking.

## Revendications

1. Procédé de fabrication d'un dispositif comportant un mécanisme de protection, comprenant :
- la mise à disposition d'un corps (101) comportant une aiguille (102) présentant un risque de blessure ;
- la mise à disposition d'au moins une feuille ;
- la mise à disposition d'un dispositif de protection (C) qui est constitué comme pièce en matière synthétique dure ou est exécuté comme première feuille (709) et deuxième feuille (711) appliquée sur le verso de la première feuille (709) avec un incision pratiquée dans la première ou la deuxième feuille (709, 711), sachant que la première feuille (709) est déformée en un renflement dans lequel une aiguille peut être engagée ;
- la liaison de l'au moins une feuille avec le dispositif de protection (C) en prévoyant une zone de pontage (B) qui est disposée entre la feuille et le mécanisme de protection (C), sachant qu'en outre des moyens sont prévus qui permettent un rabattement de la zone de pontage (B) dans la zone de transition entre une zone de liaison (A) de l'au moins une feuille et la zone de pontage (B) ;
- la fixation de l'au moins une feuille au corps (101) dans la zone de liaison (A).

2. Procédé selon la revendication 1, **caractérisé en ce que** le mécanisme de protection est fabriqué dans un matériau de départ en bande.

3. Procédé selon la revendication 2, **caractérisé en ce que** le matériau de départ en bande est enroulé sur un rouleau après la fabrication.

4. Procédé selon l'une des revendications 1 à 3, **caractérisé en ce que** le mécanisme de protection est appliqué sur le dispositif dans un procédé de distribution continu.

5. Procédé selon l'une des revendications 1 à 4, **caractérisé en ce que** la fixation de l'au moins une feuille au corps (101) est effectuée au moyen d'une colle.

6. Procédé selon l'une des revendications 1 à 5, **caractérisé en ce que** la fixation de l'au moins une feuille au corps (101) est effectuée au moyen d'une colle adhésive.

7. Procédé selon l'une des revendications 1 à 5, **caractérisé en ce que** la fixation de l'au moins une feuille au corps (101) est effectuée au moyen d'une colle adhésive permanente.

8. Procédé selon l'une des revendications 1 à 4, **caractérisé en ce que** l'au moins une feuille, pour la fixation au corps (101), est frettée sur celui-ci.

9. Mécanisme de protection pour un corps comportant une aiguille présentant un risque de blessure, comprenant :
- une zone de liaison (A) qui est constituée comme au moins une feuille et est fixée au corps (101) ;
- un dispositif de protection (C) servant à protéger contre les blessures et à loger l'aiguille (102) et constitué comme pièce en matière synthétique dure ou exécuté comme première feuille (709) et deuxième feuille (711) appliquée sur le verso de la première feuille avec un incision pratiquée dans la première ou la deuxième feuille, sachant que la première feuille (709) est déformée en un renflement dans lequel une aiguille peut être engagée ;
- une zone de pontage (B) qui est disposée entre la zone de liaison (A) et le dispositif de protection (C) ; **caractérisé par**
- des moyens qui permettent un rabattement de la zone de pontage (B) dans la zone de transition entre la zone de liaison (A) et la zone de pontage (B).

10. Mécanisme de protection selon la revendication 9, **caractérisé en ce que** l'au moins une feuille est fabriquée en PET, PE, PP ou PVC.

11. Mécanisme de protection selon la revendication 9 ou 10, **caractérisé en ce que** l'au moins une feuille présente des moyens pour l'application sur le corps (101).

12. Mécanisme de protection selon la revendication 11, **caractérisé en ce que** l'au moins une feuille est au moins en partie frettée sur le corps (101).

13. Mécanisme de protection selon la revendication 12, **caractérisé en ce que** l'au moins une feuille est frettée sous l'action de chaleur.

14. Mécanisme de protection selon la revendication 12, **caractérisé en ce que** l'au moins une feuille est frettée par action chimique ou par chaleur ou rayonnement.

15. Mécanisme de protection selon la revendication 11, **caractérisé en ce que** l'au moins une feuille est étirable et enfoncée sur le corps (101) sous forme de tuyau tout en se contractant simultanément.

16. Mécanisme de protection selon l'une des revendications 12 à 15, **caractérisé en ce que** l'au moins une feuille présente des moyens qui permettent de la déchirer dans l'état appliqué sur le corps (101).

17. Mécanisme de protection selon la revendication 16, **caractérisé en ce que** les moyens de déchirement sont constitués par une perforation.

18. Mécanisme de protection selon la revendication 16, **caractérisé en ce que** les moyens de déchirement sont constitués par un estampage.

19. Mécanisme de protection selon la revendication 16, **caractérisé en ce que** les moyens de déchirement sont constitués par une découpe.

20. Mécanisme de protection selon l'une des revendications 16 à 19, **caractérisé en ce que** les moyens de déchirement sont situés dans la zone dans laquelle l'au moins une feuille vient reposer dans la zone de l'aiguille présentant un risque de blessure dans l'état appliqué sur le corps (101).

21. Mécanisme de protection selon la revendication 20, **caractérisé en ce que** les moyens de déchirement permettent d'enlever une partie de la feuille dans la zone dans laquelle l'au moins une feuille vient reposer dans la zone de l'aiguille présentant un risque de blessure dans l'état appliqué sur le corps (101).

22. Mécanisme de protection selon la revendication 11, **caractérisé en ce qu'**une couche de colle sert de moyens d'apposition sur le corps (101).

23. Mécanisme de protection selon la revendication 22, **caractérisé en ce que** la colle est non soluble dans l'eau.

24. Mécanisme de protection selon la revendication 22 ou 23, **caractérisé en ce que** la colle est constituée par une colle adhésive permanente.

25. Mécanisme de protection selon l'une des revendications 22 à 24, **caractérisé en ce que** le plein effet de collage de la colle n'est présent que sur une surface partielle.

26. Mécanisme de protection selon la revendication 25, **caractérisé en ce que** la colle n'est appliquée que sur une surface partielle.

27. Mécanisme de protection selon la revendication 25, **caractérisé en ce que** la colle est traitée sur une surface partielle avec des moyens permettant d'atténuer ou de supprimer son effet de collage.

28. Mécanisme de protection selon l'une des revendications 9 à 27, **caractérisé en ce que** la feuille présente au moins une partie fonctionnelle supplémentaire.

29. Mécanisme de protection selon la revendication 28, **caractérisé en ce que** la partie fonctionnelle supplémentaire est constituée par au moins un coupon détachable.

30. Mécanisme de protection selon la revendication 28, **caractérisé en ce que** l'au moins une partie fonctionnelle supplémentaire est constituée par au moins un dispositif d'accrochage.

31. Mécanisme de protection selon l'une des revendications 9 à 30, **caractérisé en ce que** l'au moins une feuille présente une impression.

32. Mécanisme de protection selon la revendication 31, **caractérisé en ce que** l'impression est constituée par des informations relatives à l'utilisation du mécanisme de protection.

33. Mécanisme de protection selon l'une des revendications 9 à 32, **caractérisé en ce que** l'au moins une feuille présente une zone réimprimable.

34. Mécanisme de protection selon la revendication 33, **caractérisé en ce que** la zone de liaison (A) et la zone de pontage (B) sont constituées par la même feuille.

35. Mécanisme de protection selon la revendication 34, **caractérisé en ce que** la zone de liaison (A) et la zone de pontage (B) ainsi que la zone de protection (C) sont constituées par la même feuille.

36. Mécanisme de protection selon la revendication 33 ou 34, **caractérisé en ce que** le dispositif de protection (C) est constitué par une partie fonctionnelle fixée à au moins la zone de pontage (B).

37. Mécanisme de protection selon la revendication 9, **caractérisé en ce que** la première ou la deuxième feuille (709, 711) est constituée par une feuille déformée.

38. Mécanisme de protection selon la revendication 37, **caractérisé en ce que** la première ou la deuxième feuille (709, 711) est constituée par une feuille emboutie.

39. Mécanisme de protection selon la revendication 38, **caractérisé en ce que** la feuille emboutie est recouverte au moins sur une surface partielle par respectivement l'autre de la première et de la deuxième feuille (711, 709).

40. Mécanisme de protection selon la revendication 38 ou 39, **caractérisé en ce que** la feuille emboutie présente l'incision.

41. Mécanisme de protection selon la revendication 40, **caractérisé en ce que** l'incision ne se termine pas verticalement par rapport à un des bords de la (des) feuille(s) respective(s).

42. Mécanisme de protection selon la revendication 41, **caractérisé en ce que** l'incision présente un tracé ondulé.

43. Mécanisme de protection selon la revendication 41, **caractérisé en ce que** l'incision présente un tracé en dents de scie ou en créneaux.

44. Mécanisme de protection selon l'une des revendications 38 à 43, **caractérisé en ce que** la feuille emboutie est frettée.

45. Mécanisme de protection selon l'une des revendications 38 à 44, **caractérisé en ce que** les zones de feuille générées par l'incision se chevauchent après l'incision.

46. Mécanisme de protection selon l'une des revendications 33 à 45, **caractérisé en ce que** la zone de liaison (A) et/ou la zone de pontage (B) présentent les moyens qui permettent un rabattement défini de la zone de pontage (B) dans l'état appliqué sur le corps (110).

47. Mécanisme de protection selon la revendication 46, **caractérisé en ce que** les moyens permettant le rabattement défini sont constitués par un renforcement d'épaisseur d'au moins la zone de pontage (B).

48. Mécanisme de protection selon la revendication 47, **caractérisé en ce que** le renforcement d'épaisseur est composé d'au moins une couche de feuille supplémentaire.

49. Mécanisme de protection selon la revendication 47 ou 48, **caractérisé en ce que** le renforcement d'épaisseur est formé de parties du dispositif de protection (C).

50. Mécanisme de protection selon la revendication 46, **caractérisé en ce que** les moyens permettant le rabattement défini sont constitués par un affaiblissement au niveau de la transition entre la zone de liaison (A) et la zone de pontage (B).

51. Mécanisme de protection selon la revendication 50, **caractérisé en ce que** l'affaiblissement au niveau de la transition entre la zone de liaison (A) et la zone de pontage (B) est un découpage ou une incision.

52. Mécanisme de protection selon la revendication 50, **caractérisé en ce que** l'affaiblissement au niveau de la transition entre la zone de liaison (A) et la zone de pontage (B) est un estampage.

53. Mécanisme de protection selon la revendication 50, **caractérisé en ce que** l'affaiblissement au niveau de la transition entre la zone de liaison (A) et la zone de pontage (B) est une réduction de la largeur de la zone de pontage.

54. Mécanisme de protection selon l'une des revendications 46 à 53, **caractérisé en ce qu'**aussi bien un renforcement d'épaisseur qu'un affaiblissement au niveau de la transition entre la zone de liaison (A) et la zone de pontage (B) sont présents et que ceux-ci sont accordés l'un à l'autre dans leur effet de façon à permettre un rabattement défini.

55. Mécanisme de protection selon la revendication 36, **caractérisé en ce que** la partie fonctionnelle est constituée par une pièce moulée par injection.

56. Mécanisme de protection selon la revendication 36, **caractérisé en ce que** la partie fonctionnelle est constituée par une pièce fabriquée dans un profilé d'extrusion.

57. Mécanisme de protection selon la revendication 55 ou 56, **caractérisé en ce que** la partie fonctionnelle est constituée par une pièce en PS, PP, ABS ou TPU.

58. Mécanisme de protection selon la revendication 36, **caractérisé en ce que** la partie fonctionnelle est constituée par un composant de type peigne ou senseur.

59. Mécanisme de protection selon l'une des revendications 9 à 58, **caractérisé en ce qu'**il présente un matériau absorbant l'humidité.

60. Agencement de mécanismes de protection selon l'une des revendications 9 à 59, sachant que les mécanismes de protection sont directement ou indirectement reliés les uns aux autres.

61. Agencement selon la revendication 60, **caractérisé en ce que** les mécanismes de protection sont disposés les uns derrière les autres sous forme de tuyau.

62. Agencement selon la revendication 61, **caractérisé en ce que** les différents mécanismes de protection sont séparés les uns des autres par des affaiblissements du tuyau.

63. Agencement selon la revendication 62, **caractérisé en ce que** les affaiblissements sont constitués par des découpages en perforations.

64. Agencement selon la revendication 60, **caractérisé en ce que** les mécanismes de protection sont disposés de façon collante sur une bande support.

65. Agencement selon la revendication 64, **caractérisé en ce que** le dispositif de protection (C) des mécanismes de protection vient reposer verticalement par rapport au sens d'avancement de la bande.

66. Agencement selon la revendication 65, **caractérisé en ce qu'**un évidement de la bande support est prévu dans la zone des dispositifs de protection (C).

67. Dispositif contenant une aiguille présentant un risque de blessure, avec un mécanisme de protection selon l'une des revendications 9 à 59.

68. Dispositif selon la revendication 67, **caractérisé en ce que** le mécanisme de protection est apposé de façon autocollante sur le dispositif.

69. Dispositif selon la revendication 67, **caractérisé en ce que** le mécanisme de protection est apposé périphériquement autour du dispositif.

70. Dispositif selon la revendication 69, **caractérisé en ce que** le mécanisme de protection est fretté sur le dispositif.
